# EUROPÄISCHE PATENTANMELDUNG

(11) **EP 4 400 055 A1**
(43) Veröffentlichungstag der Anmeldung: **17.07.2024**
(21) Anmeldenummer: 23175738.6
(22) Anmeldetag: 26.05.2023
(51) Int. Cl.: A61B 6/03, A61B 6/00

(54) **COMPUTERTOMOGRAPHIE-SYSTEM UND VERFAHREN ZUM BETREIBEN EINES COMPUTERTOMOGRAPHIE-SYSTEMS**

(71) Anmelder: Siemens Healthineers AG, 91301 Forchheim (DE)
(72) Erfinder: Flohr, Thomas, 91486 Uehlfeld (DE); Stierstorfer, Karl, 91052 Erlangen (DE)
(74) Vertreter: Siemens Healthineers Patent Attorneys

(57) **Zusammenfassung**

Computertomographie-System (1) umfassend eine ringförmige Röntgenquelle (3) mit mehreren um einen Untersuchungsbereich angeordneten Teilröntgenquellen (31, 32) und einen Detektorring (4) mit mehreren um den Untersuchungsbereich angeordneten Röntgendetektoren (41), wobei das Computertomographie-System (1) dazu konfiguriert ist, während eines Computertomographie-Scans bei statischer Röntgenquelle (3) und statischem Detektorring (4) die Teilröntgenquellen (31, 32) einzeln nacheinander anzusteuern und dadurch Absorptionsprofile eines Objekts im Untersuchungsbereich aus mehreren Richtungen zu erzeugen, wobei das Computertomographie-System (1) dazu konfiguriert ist, während des Computertomographie-Scans zumindest ein erstes Röntgenspektrum mit einer ersten Gruppe der Teilröntgenquellen (31) und ein zweites von dem ersten unterscheidbares Röntgenspektrum mit einer zweiten Gruppe der Teilröntgenquellen (32) zu erzeugen und/oder dazu konfiguriert ist, mit den Röntgendetektoren (41) ein Röntgensignal spektral unterscheidbar aufzunehmen.

## Beschreibung

Die Erfindung betrifft ein Computertomographie-System und ein Verfahren zum Betreiben eines Computertomographie-Systems.

Unabhängig vom grammatikalischen Geschlecht eines bestimmten Begriffs sind Personen mit männlicher, weiblicher oder anderer Geschlechteridentität mit umfasst.

Bei Untersuchungen per Computertomographie (CT) ist es wünschenswert auch über eine rein anatomische Abbildung hinaus weitere funktionale Aussagen treffen zu können. Durch eine gleichzeitige oder nur geringfügig zeitversetzte Aufnahme von CT-Daten eines zu untersuchenden Objekts oder Subjekts, insbesondere eines Patienten, bei verschiedenen mittleren Röntgenenergien können beispielsweise Informationen über eine chemische Zusammensetzung der untersuchten Gebiete gewonnen werden. Alternativ können damit auch bestimmte Substanzen oder Materialien, z.B. jodhaltige Kontrastmittel in materialspezifischen Bildern isoliert dargestellt und quantifiziert werden. Ein bekanntes Konzept ist die Aufnahme von zwei CT-Datensätzen mit unterschiedlichen mittleren Energien, was als "Dual-Energy-CT" bezeichnet wird.

Im Stand der Technik ist die Verwendung von CT-Systemen der 3. Generation üblich. Bei diesen CT-Systemen rotieren ein Röntgenstrahler und demgegenüber ein Röntgendetektor gemeinsam um ein zu untersuchendes Subjekt. Zur Umsetzung des Dual-Energy-CT-Konzepts gibt es sogenannte Dual-Source-CT-Geräte, bei denen zwei Messsysteme der 3. Generation um 90° versetzt um das Subjekt rotieren. Die beiden Messysteme können beispielsweise gleichzeitig mit verschiedenen Beschleunigungsspannungen betrieben werden, um so gleichzeitig zwei CT-Datensätze mit unterschiedlichen mittleren Energien aufnehmen zu können.

Durch die Rotation von zwei CT-System und die dabei auftretenden Kräfte ist jedoch in der Regel eine entsprechend robuste und daher auch platzraubende Konstruktion nötig. Zudem kann durch die mechanisch nicht trivialen Anforderungen ein erhöhter Wartungsaufwand auftreten.

Es ist daher eine Aufgabe der vorliegenden Erfindung, eine Alternative für die Aufnahme verschiedener Energien bzw. spektraler Daten mit einem CT-System zu finden, die vorzugsweise mit einer mechanisch weniger aufwendigen und/oder potentiell platzsparenden Konstruktion umsetzbar ist.

Diese Aufgabe wird gelöst durch ein Computertomographie-System gemäß Anspruch 1 und ein Verfahren zum Betreiben eines Computertomographie-Systems gemäß Anspruch 15.

Gemäß einem ersten Aspekt der Erfindung ist ein Computertomographie-System vorgesehen. Das Computertomographie-System umfasst eine ringförmige Röntgenquelle mit mehreren kreisförmig oder teilkreisförmig um einen Untersuchungsbereich angeordneten Teilröntgenquellen und einen Detektorring mit mehreren kreisförmig oder teilkreisförmig um den Untersuchungsbereich angeordneten Röntgendetektoren, wobei das Computertomographie-System dazu konfiguriert ist, während eines Computertomographie-Scans bei statischer Röntgenquelle und statischem Detektorring zumindest einige der Teilröntgenquellen einzeln nacheinander anzusteuern und dadurch sowie unter Verwendung der Röntgendetektoren Absorptionsprofile eines Objekts im Untersuchungsbereich aus mehreren Richtungen zu erzeugen, wobei das Computertomographie-System dazu konfiguriert ist, während des Computertomographie-Scans zumindest ein erstes Röntgenspektrum mit einer ersten Gruppe der Teilröntgenquellen und ein zweites von dem ersten unterscheidbares Röntgenspektrum mit einer zweiten Gruppe der Teilröntgenquellen zu erzeugen und/oder dazu konfiguriert ist, mit den Röntgendetektoren ein Röntgensignal spektral unterscheidbar aufzunehmen.

Der Untersuchungsbereich kann insbesondere dazu ausgestaltet sein, dass ein zu untersuchendes Subjekt oder Objekt, insbesondere ein Patient, zumindest teilweise darin angeordnet werden kann bzw. sich darin befinden kann. Die Teilröntgenquellen können insbesondere jeweils einzelne Röntgenquellen sein, die entsprechend der Ringform der ringförmigen Röntgenquellen angeordnet sind. Es kann optional vorgesehen sein, innerhalb einer der Gruppen bzw. beider Gruppen, Teilröntgenquellen weiter in Untergruppen aufzuteilen. Die Untergruppen können jeweils mehrere Teilröntgenquellen umfassen. Optional kann zumindest eine der Untergruppen auch nur eine einzige Teilröntgenquelle umfassen. Die Röntgenquellen können vorzugsweise an unterschiedlichen Winkelpositionen der ringförmigen Röntgenquelle angeordnet sein. Die Röntgenquellen können insbesondere gleichmäßig verteilt entlang der Ringform der Röntgenquelle angeordnet sein. Vorteilhafterweise können somit Röntgenprojektionen durch den Untersuchungsreich aus verschiedenen Winkelpositionen erzeugt werden. Damit kann es insbesondere ermöglicht werden ein Computertomographiebild zu rekonstruieren entsprechend einem Bild von einem Computertomographen mit rotierendem Scanner. Die ringförmige Röntgenquelle kann eine Basisstruktur umfassen, an der bzw. entlang die Teilröntgenquellen angeordnet sind. Beispielsweise kann die ringförmige Röntgenquelle mehrere Kathoden, insbesondere winkelversetzte Kathoden, umfassen, und dazu konfiguriert sein, eine Hochspannung zwischen jeweils den Kathoden und einer Anode zu erzeugen, sodass Elektroden aus der jeweiligen Kathode emittieren, durch die Hochspannung auf die Anode gelenkt werden, dort einen Brennfleck bilden und einen Röntgenfächer erzeugen. Dieser Röntgenfächer kann vorzugsweise auf den Untersuchungsbereich gerichtet sein und von den Röntgendetektoren erfasst werden. Die Kathoden können beispielsweise Feldemissionskathoden, insbesondere Nanotubes sein. Die ringförmige Röntgenquelle kann einen vollständigen Ring oder einen Teilring bilden. Entsprechend können die Teilröntgenquellen bei einem Teilring teilkreisförmig oder bei einem vollständigen Ring kreisförmig angeordnet sein. Die Röntgendetektoren können jeweils Detektorelemente sein oder umfassen.

Vorzugsweise können die Röntgendetektoren jeweils gegenüber den Teilröntgenquellen angeordnet sein. Der Detektorring kann ein vollständiger Ring oder ein Teilring sein. Entsprechend können die Röntgendetektoren bei einem Teilring teilkreisförmig oder bei einem vollständigen Ring kreisförmig angeordnet sein. Gemäß einer Ausführungsform sind die ringförmige Röntgenquelle und der Detektorring jeweils Teilringe, die sich gegenüberliegend angeordnet sind, sodass zwischen ihnen der Untersuchungsbereich ist. Teilringe können vorteilhaft sein, wenn ein Bestrahlen nicht aus allen Richtungen notwendig ist, insbesondere um Kosten bzw. Material zu sparen bzw. um den Aufbau des Systems leichter gestalten zu können. Gemäß einer Ausführungsform sind die ringförmige Röntgenquelle und der Detektorring jeweils über einen vollständigen Ring verteilt. Bei einem vollständigen Ring kann vorteilhafterweise ein Bestrahlen des Subjekts oder Objekts aus allen Richtungen ermöglicht werden. Die ringförmige Röntgenquelle und der Detektorring können gemeinsam zu einem Ring kombiniert sein. Beispielsweise können die ringförmige Röntgenquelle und der Detektorring jeweils ein Teilring sein, die sich zu einem gemeinsamen vollständigen Ring ergänzen. Alternativ können sowohl die Röntgenquelle als auch der Detektorring sich jeweils über einen vollständigen Ring erstrecken. Beispielsweise können die Teilröntgenquellen jeweils abwechselnd mit den Röntgendetektoren in Kreisumfangsrichtung hintereinander und/oder in Radialrichtung versetzt zueinander angeordnet sein. Beispielsweise können die Teilröntgendetektoren mit einem ersten Abstand zu einem Mittelpunkt des gemeinsamen Rings und die Röntgendetektoren mit einem zweiten Abstand zu dem Mittelpunkt angeordnet sein. Alternativ oder zusätzlich können die ringförmige Röntgenquelle und der Detektorring in einer Längsrichtung parallel zu der Normalen der Kreisfläche des Rings zueinander versetzt sein. Es ist auch denkbar, dass jeweils eine(r) von Detektorring und ringförmige Röntgenquelle als Teilring und der/die andere als vollständiger Ring ausgebildet ist.

Gemäß einer ersten vorteilhaften Variante ist es vorgesehen, dass während eines Computertomographie-Scans sowohl Röntgenquelle als auch Detektorring statisch gehalten werden, das heißt insbesondere nicht um den Untersuchungsbereich rotiert werden. Statisch kann daher insbesondere so zu verstehen sein, dass bei einem Scan keine physische Drehung des Scanners um den Untersuchungsbereich erfolgt. Vorteilhafterweise kann es durch das Vermeiden einer Rotation des Scanners des CT-Systems erreicht werden, dass aufgrund der nun nicht auftretenden Rotationskräfte sowie aufgrund der somit einfacheren Anforderungen aufgrund der statischen, nichtbeweglichen Ausführung eine weniger robuste Konstruktion verwendet werden kann. Zudem kann es möglich sein, den Wartungsaufwand zu reduzieren, weil ohne Rotation ein geringerer Verschleiß möglich sein kann. Die Rotation kann dadurch entfallen, dass einige der Teilröntgenquellen einzeln nacheinander angesteuert werden können und aufgrund dessen durch die (teil- )kreisförmig angeordneten Röntgendetektoren Absorptionsprofile des Objekts im Untersuchungsbereich aus mehreren Richtungen erzeugt werden können. Beispielsweise können die Teilröntgenquellen gemäß ihrer Reihenfolge in Kreisumfangsrichtung angesteuert werden. Es ist aber auch denkbar, eine andere Reihenfolge der Ansteuerung vorzusehen. Weiterhin können durch das erfindungsgemäße System spektrale Daten aufgenommen werden. Dies kann ermöglicht werden, indem zumindest zwei verschiedene Röntgenspektren durch die zumindest zwei Gruppen von Teilröntgenquellen erzeugt werden. Unterscheidbar ist dabei insbesondere so zu verstehen, dass es möglich ist, anhand der Aufnahme mit den Röntgendetektoren der ankommenden Röntgensignale die beiden Röntgenspektren zu unterscheiden. Es kann vorgesehen sein, dass eine Energieverteilung des ersten Röntgenspektrums signifikant von einer Energieverteilung des zweiten Röntgenspektrums abweicht. Beispielsweise kann das erste Röntgenspektrum eine höhere mittlere Energie aufweisen als das zweite Röntgenspektrum oder umgekehrt. Es kann vorgesehen sein, dass ein Großteil des Energiebereichs des ersten Röntgenspektrums nicht mit einem Großteil des Energiebereichs des zweiten Röntgenspektrums überlappt. Ein Großteil kann mehr als fünfzig Prozent, vorzugsweise mehr als 60 Prozent, besonders bevorzugt mehr als 75 Prozent, des jeweiligen Röntgenspektrums sein. Es kann vorgesehen sein, dass zuerst die erste Gruppe von Teilröntgenquellen angesteuert bzw. betrieben wird und danach die zweite Gruppe von Teilröntgenquellen betrieben wird. Es kann vorgesehen sein, mehr als zwei Gruppen von Teilröntgenquellen hintereinander anzusteuern.

Alternativ kann gemäß einer zweiten Variante das Aufnehmen von spektralen Daten dadurch ermöglicht werden, dass die Röntgendetektoren ein Röntgensignal spektral unterscheidbar aufnehmen können. Beispielsweise kann es vorgesehen sein, dass alle Teilröntgenquellen, ein im Wesentlichen gleiches Spektrum erzeugen und dass dieses Spektrum automatisch in zwei Teilspektren aufteilen, z.B. jeweils eines oberhalb und eines unterhalb einem festgesetzten Energieschwellwert. Mit Vorteil kann somit eine spektrale Erfassung durch die Teilröntgenquellen ermöglicht werden.

Es ist aber auch denkbar, diese beiden Varianten in einer dritten Variante zu kombinieren. Entsprechend, kann es beispielsweise vorgesehen sein, dass eine die Gruppe von Teilröntgenquellen das erste Röntgenspektrum aufnimmt und die zweite Gruppe von Teilröntgenquellen das zweite Röntgenspektrum aufnimmt, wobei die Röntgendetektoren die ankommenden Röntgensignale spektral unterscheidbar aufzunehmen. Beispielsweise kann somit die Unterscheidbarkeit zwischen den zumindest zwei Röntgenspektren bzw. eine spektrale Trennung verbessert werden. Es kann aber auch vorgesehen sein, durch die Kombination eine Aufteilung in noch mehr unterscheidbare Spektren zu erreichen. Beispielsweise kann das System dazu konfiguriert sein, dass das erste Röntgenspektrum und das zweite Röntgenspektrum jeweils noch einmal durch die Röntgendetektoren in zwei Unterspektren aufgeteilt werden. Somit kann vorteilhafterweise eine größere Anzahl an verschiedenen Spektren, insbesondere mit jeweils verschiedenen mittleren Energien, erzielt werden. Beispielsweise können die Röntgendetektoren dazu konfiguriert sein, vier verschiedene Energiebereiche zu unterscheiden und es können zwei Gruppen von Teilröntgenquellen und somit zwei von der ringförmigen Röntgenquelle erzeugte Röntgenspektren vorgesehen sein. Vorteilhafterweise können in diesem Beispiel Computertomographiedaten mit acht (4 mal 2) verschiedenen mittleren Energien ermöglicht werden.

Gemäß einer Ausführungsform ist das Computertomographie-System dazu konfiguriert, das erste Röntgenspektrum durch Anlegen einer ersten Beschleunigungsspannung bei den Teilröntgenquellen der ersten Gruppe der Teilröntgenquellen und das zweite Röntgenspektrum durch Anlegen einer zweiten Beschleunigungsspannung bei den Teilröntgenquellen der zweiten Gruppe der Teilröntgenquellen zu erzeugen. Die erste bzw. zweite Beschleunigungsspannung wird insbesondere zwischen einer Kathode der jeweiligen Teilröntgenquelle und einer dazugehörigen Anode angelegt. Es hat sich gezeigt, dass mit zwei Beschleunigungsspannungen (oder optional mehr) gemäß dieser Ausführungsform eine gute spektrale Unterscheidbarkeit erreicht werden kann. Beispielsweise kann die erste Beschleunigungsspannung im Bereich von 50 kV bis 100 kV liegen, bevorzugt im Bereich von 70 kV bis 90 kV. Beispielsweise kann die zweite Beschleunigungsspannung im Bereich von 100 bis 200 kV, bevorzugt im Bereich von 120 kV bis 170 kV, besonders bevorzugt im Bereich von 130 kV bis 150 kV, liegen. Ein konkretes Beispiel kann eine erste Beschleunigungsspannung von 80 kV und eine zweite Beschleunigungsspannung von 140 kV sein. Es hat sich gezeigt, dass mit solchen Werten besonders gute Ergebnisse erzielt werden können, insbesondere indem die Spektren gut unterscheidbar sein können und gleichzeitig in einem für die Bildgebung günstigen Bereich liegen. Beispielsweise kann es vorgesehen sein, dass nacheinander verschieden Teilröntgenquellen angesteuert werden, wobei beim Umschalten von einer Teilröntgenquelle (bzw. Kathode) oder Untergruppe (bzw. Kathodengruppe) zur nächsten auch die Beschleunigungsspannung umgeschaltet wird. Damit können insbesondere zeitlich aufeinanderfolgende Röntgenprojektionen mit unterschiedlichem Röntgenspektrum bzw. unterschiedlicher mittlerer Röntgenenergie (mit Hilfe der Röntgendetektoren) aufgenommen werden. Alternativ ist es auch denkbar, dass mehrere aufeinanderfolgend angesteuerte Teilröntgenquellen mit jeweils der gleichen Beschleunigungsspannung angesteuert werden und die Beschleunigungsspannung nach einer vorbestimmten Anzahl an Umschaltungen geändert wird. Mit anderen Worten kann das System dazu konfiguriert sein, aufeinanderfolgend Teilröntgenquellen der gleichen Gruppe (z.B. nur der ersten Gruppe) anzusteuern und daraufhin aufeinanderfolgend Teilröntgenquellen der anderen Gruppe (z.B. nur der zweiten Gruppe) anzusteuern. Bei zwei Gruppen kann das Computertomographie-System insbesondere ein Dual-Energy-System sein.

Gemäß einer Ausführungsform umfasst die ringförmige Röntgenquelle eine erste Spannungsbeschaltung zum Erzeugen einer Beschleunigungsspannung für die ersten Gruppe der Teilröntgenquellen und eine zweite Spannungsbeschaltung zum Erzeugen einer Beschleunigungsspannung für die zweite Gruppe der Teilröntgenquellen. Optional kann die Röntgenquelle eine oder mehrere weitere Spannungsbeschaltungen für eine oder mehrere weitere Gruppen umfassen. Die Spannungsbeschaltungen können vorzugsweise kreisförmig bzw. teilkreisförmig entsprechend der ringförmigen Form der Röntgenquelle ausgebildet sein. Beispielseise kann die erste Spannungsbeschaltung dazu ausgestaltet sein, die erste Beschleunigungsspannung wie hierin beschrieben zu erzeugen und die die zweite Spannungsbeschaltung kann dazu ausgestaltet sein, die zweite Beschleunigungsspannung wie hierin beschrieben zu erzeugen. Gemäß einer Ausführungsform sind die erste und die zweite Spannungsbeschaltung dazu konfiguriert, die Teilröntgenquellen jeweils einzeln oder in Untergruppen mit eine Beschleunigungsspannung zu versorgen.

Gemäß einer Ausführungsform ist das Computertomographie-System dazu konfiguriert, jeweils abwechselnd zumindest eine der Teilröntgenquellen der ersten Gruppe und zumindest eine der Teilröntgenquellen der zweiten Gruppe anzusteuern. Das System kann dazu konfiguriert sein, jeweils alternierend bzw. abwechselnd nacheinander Untergruppen der ersten Gruppe und Untergruppen der zweiten Gruppe anzusteuern. Beispielsweise kann das System dazu konfiguriert sein, jeweils abwechselnd eine Teilröntgenquelle der ersten Gruppe und eine Teilröntgenquelle der zweiten Gruppe anzusteuern, insbesondere so, dass nacheinander alle Teilröntgenquellen angesteuert wurden. Beispielsweise kann die erste Gruppe mehrere Untergruppen von Teilröntgenquellen umfassen. Zumindest einige der Untergruppen bestehen vorzugsweise aus jeweils mehreren Teilröntgenquellen. Beispielsweise kann das System dazu konfiguriert sein, jeweils abwechselnd eine Untergruppe der ersten Gruppe und eine Untergruppe der zweiten Gruppe anzusteuern, insbesondere so, dass nacheinander alle Untergruppen angesteuert wurden. Optional kann zumindest eine der Untergruppen aus einer einzelnen Teilröntgenquelle bestehen. Das System kann dazu konfiguriert sein, jeweils abwechselnd eine Teilröntgenquelle oder eine Untergruppe der ersten Gruppe mit der ersten Beschleunigungsspannung und eine Teilröntgenquelle oder eine Untergruppe der zweiten Gruppe mit der zweiten Beschleunigungsspannung anzusteuern. Das Ansteuern erfolgt insbesondere durch Anlegen der ersten bzw. zweiten Beschleunigungsspannung.

Gemäß einer Ausführungsform sind die Teilröntgenquellen auf der ringförmigen Röntgenquelle derart angeordnet, dass in Umfangsrichtung jeweils abwechselnd eine Teilröntgenquelle der ersten Gruppe und eine Teilröntgenquelle der zweiten Gruppe angeordnet ist. Vorteilhafterweise kann es somit möglich sein, eine besonders gute räumliche Aufteilung der zumindest zwei verschiedenen Röntgenspektren zu erzielen. Optional ist das Computertomographie-System zudem dazu konfiguriert, die Teilröntgenquellen in der Reihenfolge ihrer Anordnung auf dem Kreisumfang anzusteuern. Vorteilhafterweise kann es somit möglich sein, eine besonders gute zeitliche Aufteilung der zumindest zwei verschiedenen Röntgenspektren zu erzielen. Damit können ggf. Fehlern bei einer spektralen Analyse in Folge von Bewegung des Subjekts oder Objekts vermindert werden.

Gemäß einer Ausführungsform ist das Computertomographie-System dazu ausgestaltet, das zweite Röntgenspektrum zu erzeugen, indem die von der zweiten Gruppe von Teilröntgenquellen erzeugte Röntgenstrahlung mit Röntgenfiltern angepasst wird. Vorzugsweise sind für alle Teilröntgenquellen der zweiten Gruppe eine gleiche Art von Filtern vorgesehen. Eine gleiche Art bezieht sich dabei insbesondere auf die Filtereigenschaften im Röntgenspektrum. Die Röntgenfilter sind vorzugsweise dazu ausgestaltet, bestimmte Energien, insbesondere einen Energiebereich, aus dem Spektrum herauszufiltern oder zumindest abzuschwächen. Vorzugsweise können die Röntgenfilter dazu ausgestaltet sein Röntgenenergien, die unterhalb einer Energieschwelle, liegen herauszufiltern. Mit anderen Worten können die Röntgenfilter dazu ausgestaltet sein, das Röntgenspektrum aufzuhärten. Durch das Vorsehen der Röntgenfilter können somit zwei unterscheidbare Röntgenspektren erzeugt werden. Diese Variante stellt eine besonders günstige Alternative dar, insbesondere, weil nicht notwendigerweise eine zweifache Spannungsbeschaltung bereitgestellt werden muss. Es kann vorteilhafterweise vorgesehen sein, diese Ausführungsform mit der Ausführungsform mit einer ersten und einer zweiten Beschleunigungsspannung zu kombinieren. Es hat sich gezeigt, dass die spektrale Trennung, welche durch zwei Beschleunigungsspannungen bereits recht gut erzielt werden kann, durch das zusätzliche Verwenden der Röntgenfilter weiter verbessert werden kann. Typischerweise wird durch die Beschleunigungsspannungen ein Energiespektrum erzeugt. Eine maximale Energie ist typischerweise durch die Beschleunigungsspannung definiert, während jedoch ein Energiebereich, indem eine Anzahl an Photonen nicht vernachlässigbar ist deutlich niedriger liegen kann. Beispielsweise kann das erzeugte Röntgenspektrum einer Beschleunigungsspannung von 140 kV unter Umständen bis hin zu 30 kV relevante Anteile aufweisen. Die breite des tatsächlich gemessenen Röntgenspektrums kann auch von einer Empfindlichkeit der Röntgendetektoren abhängen. Entsprechend kann ein Röntgenspektrum relativ breit ausfallen. Dadurch kann es in der Praxis zu einem Überlapp der beiden erzeugten Röntgenspektren kommen. Durch das Vorsehen der Röntgenfilter, insbesondere Filter vor der zweiten Gruppe mit höherer Beschleunigungsspannung, kann das jeweilige Röntgenspektrum entsprechend begrenzt werden, um einen Überlapp der beiden Spektren zu vermeiden oder zumindest zu verringern. Bei dieser Kombination kann ein Filter vor den Teilröntgenquellen der zweiten Gruppe besonders vorteilhaft sein, wenn er Röntgenenergien, die unterhalb einer Energieschwelle, liegen, herausfiltert. Vorzugsweise kann die Energieschwelle der Röntgenfilter zwischen der der ersten Beschleunigungsspannung entsprechenden Energie und der der zweiten Beschleunigungsspannung entsprechenden Energie liegen. Bei Beschleunigungsspannungen von 80 kV und 140 kV könnte die Energieschwelle demnach vorzugsweise im Bereich zwischen 80 keV und 140 keV liegen. Die Röntgenfilter können beispielsweise Zinnfilter sein. Zinnfilter können aufgrund ihrer Filtereigenschaften besonders geeignet sein, für das Filter der zweiten Gruppe von Teilröntgenquellen. Entsprechend der Anordnung der Teilröntgenquellen der ersten Gruppe und der zweiten Gruppe können die Röntgenfilter so angeordnet sein, dass z.B. in Kreisumfangsrichtung jede zweite Teilröntgenquelle mit einem Röntgenfilter ausgestattet ist. Bei alternativer Anordnung der Teilröntgenquellen der ersten Gruppe und der zweiten Gruppe können beispielsweise mehrere benachbarte Teilröntgenquellen mit einem Röntgenfilter versehen sein. Die Röntgenfilter können optional derart ausgestaltet sein, dass ein erster Teil der ausgestrahlten Röntgenstrahlung einer Röntgenquelle durch einen ersten Filterteil gefiltert wird und ein zweiter Teil mit einem zweiten Filterteil oder gar nicht gefiltert wird, wobei sich die räumliche Ausbreitung des ersten Teils und des zweiten Teils derart unterscheidet, dass der erste Teil von anderen der Röntgendetektoren oder Detektorelemente erfasst wird als der zweite Teil. Die Röntgendetektoren können beispielsweise Mehrzeilendetektoren mit zeilenweise Detektorelementen sein. Beispielsweise kann eine Hälfte, d.h. insbesondere eine Hälfte von Detektorelementen, eines Mehrzeilendetektors ein Röntgenspektrum mit höherer mittlerer Energie erfassen als die andere Hälfte, d.h. insbesondere die andere Hälfte von Detektorelementen, des Detektors. Hierdurch kann eine weitere spektrale Aufteilung möglich sein.

Gemäß einer Ausführungsform ist das Computertomographie-System dazu ausgestaltet, das erste Röntgenspektrum zu erzeugen, indem die von der ersten Gruppe von Teilröntgenquellen erzeugte Röntgenstrahlung ebenfalls mit weiteren Röntgenfiltern angepasst wird. Die weiteren Röntgenfilter können sich vorzugsweise von den Röntgenfiltern der zweiten Gruppe hinsichtlich ihrer Filtereigenschaften unterscheiden. Vorzugsweise können die weiteren Röntgenfilter dazu ausgestaltet sein, eine mittlere Energie des Röntgenspektrums hin zu niedrigeren Werten zu verschieben. Beispielsweise können die weiteren Röntgenfilter Goldfilter sein. Die weiteren Röntgenfilter (zusätzlich zu den Röntgenfiltern vor den Teilröntgenquellen der zweiten Gruppe) können die spektrale Unterscheidbarkeit weiter verbessern. Auch diese weitergehende Ausführungsform kann vorteilhafterweise mit der Ausführungsform mit einer ersten und einer zweiten Beschleunigungsspannung kombiniert werden, insbesondere um die spektrale Unterscheidbarkeit noch weiter zu verbessern.

Gemäß einer Ausführungsform umfasst die ringförmige Röntgenquelle zumindest vor der zweiten Gruppe von Teilröntgenquellen Filterslots zum manuellen oder automatischen Einsetzen von Röntgenfiltern. Alternativ oder zusätzlich kann die ringförmige Röntgenquelle zumindest vor der zweiten Gruppe von Teilröntgenquellen Röntgenfilter umfassen. Vorzugsweise sind die Filterslots derart ausgestaltet, dass die Röntgenfilter, wenn sie in den Filterslots eingesetzt sind, sich vor den entsprechenden Teilröntgenquellen befinden. Die Filterslots können ein einfaches Austauschen der Filter ermöglichen. Beispielsweise, um verschiedene Röntgenspektren erzeugen zu können. Das Computertomographie-System kann für jeden der Filterslots eine, insbesondere automatische, Einfahrmechanik für die Röntgenfilter umfassen. Das System kann beispielsweise dazu ausgestaltet sein, die Filter derart aus ihrer Position vor den Teilröntgenquellen herauszufahren, dass sie von einem Nutzer entnommen bzw. ausgetauscht werden können. Das System kann einen Filterlagerbereich umfassen, in dem die Filter gelagert werden können, solange sie sich nicht vor den Teilröntgenquellen befinden. Die Einfahrmechanik kann dazu ausgestaltet sein, die Röntgenfilter von den Filterslots in den Filterlagerbereich und zurück zu bewegen.

Gemäß einer Ausführungsform ist das Computertomographie-System dazu konfiguriert, während des Computertomographie-Scans eine oder mehrere weitere Röntgenspektren mit einer oder mehreren weiteren Gruppen, darunter zumindest einer dritten Gruppe, der Teilröntgenquellen zu erzeugen. Mit anderen Worten kann das System dazu konfiguriert sein, mehr als zwei Gruppen mit jeweils einer eigenen Beschleunigungsspannung und einem eigenen Röntgenspektrum anzusteuern. Beispielsweise kann das System dazu konfiguriert sein, zusätzlich zumindest ein drittes Röntgenspektrum durch Anlegen einer dritten Beschleunigungsspannung an eine dritte Gruppe der Teilröntgenquellen zu erzeugen. Vorteilhafterweise können mit dieser Ausführungsform mehr als zwei Energien spektral auflösbar sein.

Gemäß einer Ausführungsform umfasst der Detektorring photonenzählende Detektoren, die dazu konfiguriert sind, zumindest zwei Energieschwellen zu unterscheiden. Vorteilhafterweise können somit photonenzählende Detektoren verwendet werden, um eine Erzeugung von spektralen Daten zu ermöglichen. Die Detektoren können vorzugsweise dazu ausgestaltet sein, aus erfasster Röntgenstrahlung jeweils zwei Datensätze zu erzeugen, wobei ein erster Datensatz Energien oberhalb eines ersten Schwellwertes umfasst und ein zweiter Datensatz Energien oberhalb eines zweiten Schwellwertes, der größer ist als der erste Schwellwert, umfasst.

Gemäß einer Ausführungsform sind die Röntgendetektoren als Detektorpaare vorgesehen, von denen einer im Wesentlichen nieder-energetische Röntgenstrahlung und der andere im Wesentlichen hochenergetische Röntgenstrahlung absorbiert. Insbesondere können die Röntgendetektoren Dual-Layer-Detektoren sein. Dual-Layer-Detektoren bestehen insbesondere aus zwei in Strahlrichtung übereinander angeordneten Detektoren, wobei der obere, dem Röntgenstrahler nähere Detektor primär nieder-energetische Röntgenquanten absorbiert, der untere die verbleibenden höherenergetischen Röntgenquanten. Die photonenzählenden Detektoren oder die Detektorpaare können mit der Ausführungsform kombiniert werden, gemäß der ein erstes Röntgenspektren und ein zweites Röntgenspektrum von zwei Gruppen von Teilröntgenquellen erzeugt werden, insbesondere mit der speziellen Ausführungsform mit zumindest zwei verschiedenen Beschleunigungsspannungen. Das System kann dazu konfiguriert sein, die Schwellenwerte der Detektoren dynamisch einzustellen und mit dem Anlegen der Beschleunigungsspannungen zu synchronisieren. Die Kombination kann eine spektrale Trennung bzw. Unterscheidbarkeit weiter verbessern. Alternativ kann es möglich sein, eine größere Anzahl spektral unterscheidbarer Röntgendaten zu erzeugen. Beispielsweise kann die Kombination von photonenzählenden Detektoren mit vier Energieschwellen mit Spannungsumschaltung zwischen zwei verschiedenen Beschleunigungsspannungen, entsprechend den zwei Gruppen, zu Computertomographie-Daten mit acht verschiedenen mittleren Energien führen.

Ein weiterer Aspekt der Erfindung ist ein Verfahren zum Betreiben eines Computertomographie-Systems mit mehreren Teilröntgenquellen und mehreren Röntgendetektoren, die jeweils örtlich unbeweglich betrieben werden und einen Untersuchungsbereich umgeben, umfassend die folgenden Schritte:
(a) Ansteuern einer ersten Teilröntgenquelle oder einer ersten Untergruppe von Teilröntgenquellen, sodass eine auf den Untersuchungsbereich gerichtete Röntgenstrahlung mit einem ersten Röntgenspektrum erzeugt wird;
(b) Ansteuern einer zweiten Teilröntgenquelle oder einer zweiten Untergruppe von Teilröntgenquellen, sodass eine auf den Untersuchungsbereich gerichtete Röntgenstrahlung mit einem zweiten von dem ersten verschiedenen Röntgenspektrum erzeugt wird;
(c) Detektieren der durch den Untersuchungsbereich gelangten Röntgenstahlen des ersten und des zweiten Röntgenspektrums mit den Röntgendetektoren.

Vorzugsweise können die Schritte a und b zeitversetzt durchgeführt werden. Besonders bevorzugt können die Schritte a, b und c mehrfach wiederholt werden, wobei in den Schritten a und b jeweils anderen Teilröntgenquellen bzw. Untergruppen angesteuert werden. Insbesondere können in Kreisumfangsrichtung reihum Teilröntgenquellen oder Untergruppen jeweils abwechselnd gemäß Schritt a und Schritt b angesteuert werden. Vorzugsweise sind die Teilröntgenquellen und die Röntgendetektoren kreisförmig um den Untersuchungsbereich verteilt, insbesondere derart, dass jeweils einzelne Röntgendetektoren jeweils einzelnen Teilröntgenquellen gegenüber angeordnet sind. Alle Vorteile und Merkmale des Systems können analog auf das Verfahren übertragen werden und umgekehrt.

Alle hierin beschriebenen Ausführungsformen können miteinander kombiniert werden, soweit nicht explizit etwas anderes angegeben ist.

Im Folgenden werden Ausführungsformen mit Bezug auf die beigefügten Figuren beschrieben.
- Fig. 1: zeigt ein Computertomographie-System gemäß einer Ausführungsform der Erfindung,
- Fig. 2: zeigt eine ringförmigen Röntgenquelle und einen Detektorring eines Computertomographie-Systems gemäß einer Ausführungsform der Erfindung,
- Fig. 3: zeigt eine ringförmigen Röntgenquelle und einen Detektorring eines Computertomographie-Systems gemäß einer weiteren Ausführungsform der Erfindung,
- Fig. 4: zeigt eine ringförmigen Röntgenquelle und einen Detektorring eines Computertomographie-Systems gemäß einer weiteren Ausführungsform der Erfindung,
- Fig. 5: zeigt eine ringförmigen Röntgenquelle und einen Detektorring eines Computertomographie-Systems gemäß einer weiteren Ausführungsform der Erfindung,
- Fig. 6: zeigt eine ringförmigen Röntgenquelle und einen Detektorring eines Computertomographie-Systems gemäß einer weiteren Ausführungsform der Erfindung
- Fig. 7: zeigt eine ringförmigen Röntgenquelle und einen Detektorring eines Computertomographie-Systems gemäß einer weiteren Ausführungsform der Erfindung und
- Fig. 8: zeigt ein Flussdiagramm eines Verfahrens zum Betreiben eines Computertomographie-Systems gemäß einer Ausführungsform der Erfindung.

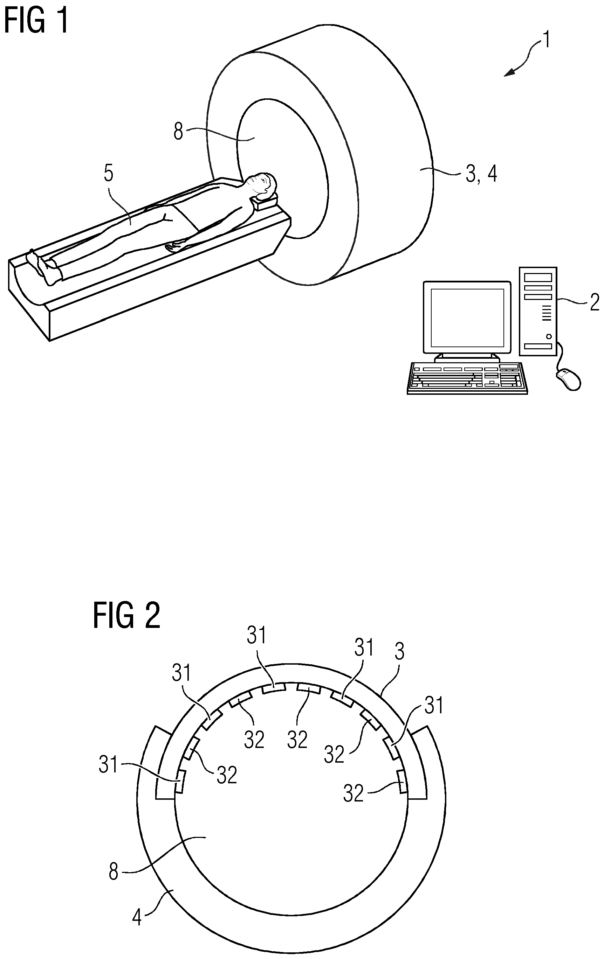

Figur 1 zeigt ein Computertomographie-System 1 gemäß einer Ausführungsform der Erfindung. Das System 1 umfasst einen Untersuchungsbereich 8, um den herum eine ringförmig Röntgenquelle 3 und ein Detektorring 4 angeordnet sind. Zum Zwecke eines Computertomographie-Scans kann ein Subjekt 5, insbesondere ein Patient, in den Untersuchungsbereich 8 gefahren werden. Das Computertomographie-System 1 umfasst eine Steuereinheit 2, z.B. einen Computer, die dazu konfiguriert ist, das System 1 zu steuern. Die Röntgenquelle 3 und der Detektorring 4 können beispielsweise so ausgestaltet sein, wie in den folgenden Figuren 2 bis 5 gezeigt.

Figur 2 zeigt eine ringförmigen Röntgenquelle 3 und einen Detektorring 4 eines Computertomographie-Systems 1 gemäß einer Ausführungsform der Erfindung. Die ringförmigen Röntgenquelle 3 und der Detektorring 4 sind jeweils als Teilring ausgestaltet und um einen Untersuchungsbereich 8 angeordnet. Dabei sind teilkreisförmig entlang dem Detektorring 4 angeordnete Röntgendetektoren 41 (hier nicht gezeigt) gegenüber teilkreisförmig angeordneten Teilröntgenquellen 31, 32 der ringförmigen Röntgenquelle 3 platziert. Das System 1 ist dazu konfiguriert, während eines Computertomographie-Scans ein erstes Röntgenspektrum mit einer ersten Gruppe der Teilröntgenquellen 31 zu erzeugen und ein zweites vom ersten unterscheidbares Röntgenspektrum mit einer zweiten Gruppe der Teilröntgenquellen 32 zu erzeugen. Das Computertomographie-System 1 kann optional dazu konfiguriert sein, jeweils abwechselnd zumindest eine der Teilröntgenquellen 31 der ersten Gruppe und zumindest eine der Teilröntgenquellen 32 der zweiten Gruppe anzusteuern. Bei der hier gezeigten Anordnung der Teilröntgenquellen 31, 32, wonach in Kreisumfangsrichtung jeweils abwechselnd Teilröntgenquellen 31 der ersten Gruppe und Teilröntgenquellen 32 der zweiten Gruppe vorgesehen sind, kann das System 1 somit insbesondere dazu konfiguriert sein, die Teilröntgenquellen 31, 32 in der Reihenfolge ihrer Anordnung auf dem Kreisumfang anzusteuern.

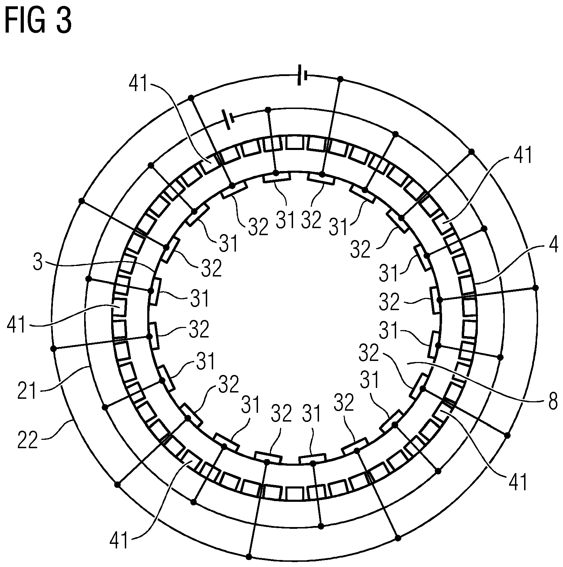

Figur 3 zeigt eine ringförmigen Röntgenquelle 3 und einen Detektorring 4 eines Computertomographie-Systems 1 gemäß einer weiteren Ausführungsform der Erfindung. Die ringförmige Röntgenquelle 3 und der Detektorring 4 sind jeweils über einen vollständigen Ring verteilt. Die ringförmige Röntgenquelle 3 umfasst mehrere kreisförmig um einen Untersuchungsbereich 8 angeordnete Teilröntgenquellen 31, 32. Der Detektorring 4 umfasst mehrere kreisförmig um den Untersuchungsbereich 8 angeordneten Röntgendetektoren 41, die jeweils gegenüber den Teilröntgenquellen 31, 32 angeordnet sind. Vorzugsweise sind in diesen und den folgenden Ausführungsformen die Teilröntgenquellen 31, 32 und die Röntgendetektoren 42 in Achsrichtung, d.h. in eine in die Bildebenen senkrecht hineinzeigende Richtung zueinander versetzt. Das System 1 ist dazu konfiguriert, während eines Computertomographie-Scans ein erstes Röntgenspektrum mit einer ersten Gruppe der Teilröntgenquellen 31 zu erzeugen und ein zweites vom ersten unterscheidbares Röntgenspektrum mit einer zweiten Gruppe der Teilröntgenquellen 32 zu erzeugen. Das erste und das zweite Röntgenspektrum können insbesondere verschiedene mittlere Energien aufweisen. Während der Aufnahme erfolgt insbesondere keine Rotation der Röntgenquelle 3 oder des Detektorrings 4. Die Aufnahme wird vorzugsweise durchgeführt, indem durch ein Ansteuern von einzelnen der in verschiedenen Winkeln angeordneten Teilröntgenquellen 31, 32 im Zusammenspiel mit den Röntgendetektoren 41 Absorptionsprofile eines Subjekts 5 oder Objekts im Untersuchungsbereich 8 aus mehreren Richtungen erzeugt werden. Dazu umfasst die ringförmige Röntgenquelle 3 eine erste Spannungsbeschaltung 21 zum Erzeugen einer Beschleunigungsspannung für die ersten Gruppe der Teilröntgenquellen 31 und eine zweite Spannungsbeschaltung 22 zum Erzeugen einer Beschleunigungsspannung für die zweite Gruppe der Teilröntgenquellen 32. Durch Anlegen der ersten Beschleunigungsspannung bei den Teilröntgenquellen 31 der ersten Gruppe wird das erste Röntgenspektrum erzeugt und das zweite Röntgenspektrum wird durch Anlegen einer zweiten Beschleunigungsspannung bei den Teilröntgenquellen 32 der zweiten Gruppe erzeugt. Die Teilröntgenquellen 31, 32 können insbesondere in der Reihenfolge ihrer Anordnung auf dem Kreisumfang der ringförmigen Röntgenquelle 3 angesteuert werden. Beispielsweise können durch ein schnelles Hin- und Herschalten zwischen den zwei Beschleunigungsspannungen aufeinanderfolgende CT-Projektionen mit jeweils abwechselnd dem ersten Röntgenspektrum und dem zweiten Röntgenspektrum aufgenommen werden. Alternativ, falls die Aufnahme der CT-Daten bei einem unbewegten Subjekt 5 oder Objekt erfolgt, d.h. ohne zeitliche Änderungen z. B. einer Kontrastmittelkonzentration, kann das Hin- und Herschalten zwischen den zwei Beschleunigungsspannungen optional auch langsamer erfolgen. Beispielsweise können jeweils mehrere Projektionen mit der einen und dann mehrere Projektionen mit der anderen Beschleunigungsspannung aufgenommen werden. Optional kann das System 1 weiterhin dazu konfiguriert sein, während des Computertomographie-Scans mit den Röntgendetektoren 41 ein Röntgensignal spektral unterscheidbar aufzunehmen. Die Röntgendetektoren 41 können beispielsweise photonenzählende Detektoren sein, die dazu konfiguriert sind, zumindest zwei Energieschwellen zu unterscheiden. Durch diese Kombination kann die spektrale Trennung noch weiter verbessert werden. Alternativ oder zusätzlich kann durch diese Kombination zudem die Anzahl der unterscheidbaren Röntgenspektren erhöht werden, beispielsweise auf insgesamt vier, bei zwei Energieschwellen der Röntgendetektoren 41.

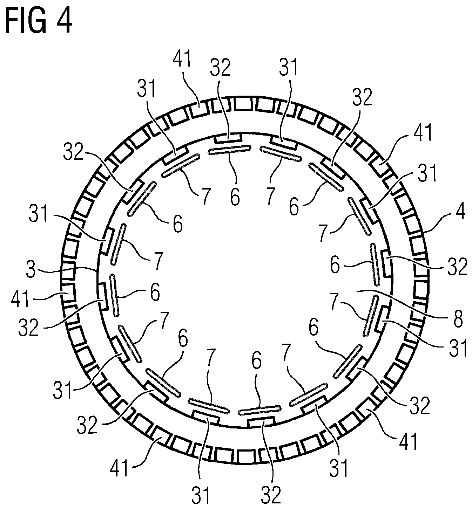

Figur 4 zeigt eine ringförmigen Röntgenquelle 3 und einen Detektorring 4 eines Computertomographie-Systems 1 gemäß einer weiteren Ausführungsform der Erfindung. Die ringförmige Röntgenquelle 3 und der Detektorring 4 sind jeweils über einen vollständigen Ring verteilt. Die ringförmige Röntgenquelle 3 umfasst mehrere kreisförmig um einen Untersuchungsbereich 8 angeordnete Teilröntgenquellen 31, 32. Der Detektorring 4 umfasst mehrere kreisförmig um den Untersuchungsbereich 8 angeordneten Röntgendetektoren 41, die jeweils gegenüber den Teilröntgenquellen 31, 32 angeordnet sind. Das Computertomographie-System 1 ist dazu ausgestaltet, das zweite Röntgenspektrum unterschiedlich von dem ersten Röntgenspektrum zu erzeugen, indem die von der zweiten Gruppe von Teilröntgenquellen 32 erzeugte Röntgenstrahlung mit Röntgenfiltern 6 angepasst wird. Das bedeutet, dass insbesondere die Beschleunigungsspannung aller Teilröntgenquellen 31, 32 gleich sein kann und die Unterscheidbarkeit einzig durch die Röntgenfilter 6 verursacht werden kann. Optional können auch vor der ersten Gruppe von Teilröntgenquellen 31 jeweils weitere Röntgenfilter 7 vorgesehen sein, welche sich von den Röntgenfiltern 6 der zweiten Gruppe hinsichtlich ihrer Filtereigenschaften unterscheiden. Damit kann eine noch bessere Unterscheidbarkeit der zwei Röntgenspektren erreichbar sein. Für die Röntgenfilter 6, 7 können Filterslots zum manuellen oder automatischen Einsetzen der Röntgenfilter 6, 7 vorgesehen sein. Diese können grundsätzlich in allen Ausführungsformen, in denen Röntgenfilter 6, 7 zum Einsatz kommen, verwendet werden.

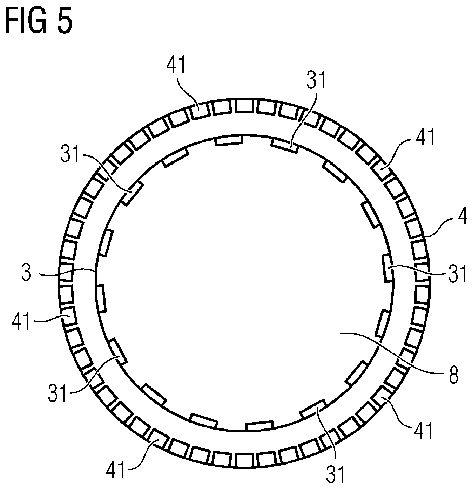

Figur 5 zeigt eine ringförmigen Röntgenquelle 3 und einen Detektorring 4 eines Computertomographie-Systems 1 gemäß einer weiteren Ausführungsform der Erfindung. Die ringförmige Röntgenquelle 3 und der Detektorring 4 sind jeweils über einen vollständigen Ring verteilt. Die ringförmige Röntgenquelle 3 umfasst mehrere kreisförmig um einen Untersuchungsbereich 8 angeordnete Teilröntgenquellen 31, 32. Der Detektorring 4 umfasst mehrere kreisförmig um den Untersuchungsbereich 8 angeordneten Röntgendetektoren 41, die jeweils gegenüber den Teilröntgenquellen 31, 32 angeordnet sind. Das Computertomographie-System 1 ist dazu konfiguriert, während eines Computertomographie-Scans mit den Röntgendetektoren 41 ein Röntgensignal spektral unterscheidbar aufzunehmen und somit spektrale Daten zu erzeugen. Dazu können die Röntgendetektoren 41 beispielsweise photonenzählende Detektoren sein, die dazu konfiguriert sind, zumindest zwei Energieschwellen zu unterscheiden und somit zwei unterscheidbare Röntgenspektren aufzunehmen.

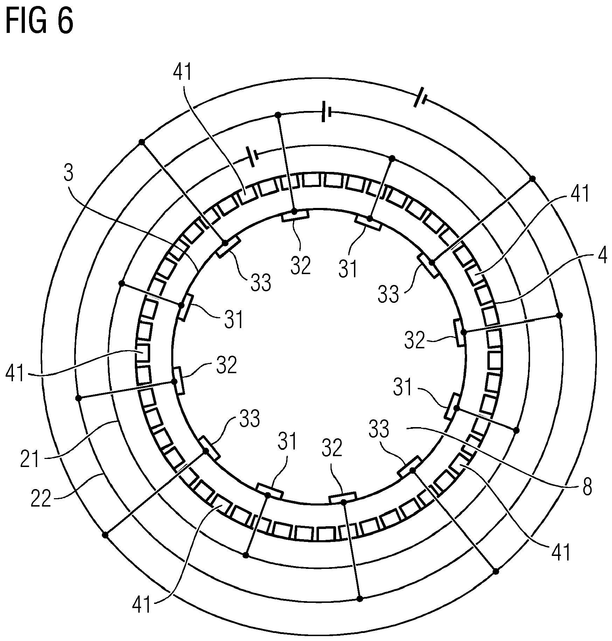

Figur 6 zeigt eine ringförmigen Röntgenquelle 3 und einen Detektorring 4 eines Computertomographie-Systems 1 gemäß einer weiteren Ausführungsform der Erfindung. Diese Ausführungsform kann in wesentlichen Punkten der in Figur 3 gezeigten entsprechen, unterscheidet sich jedoch dadurch, dass das Computertomographie-System 1 dazu konfiguriert ist, während des Computertomographie-Scans ein weiteres Röntgenspektrum mit einer dritten Gruppe von Teilröntgenquellen 33 zu erzeugen. Dadurch können spektrale Daten mit drei verschiedenen Energiestufen erzeugt werden.

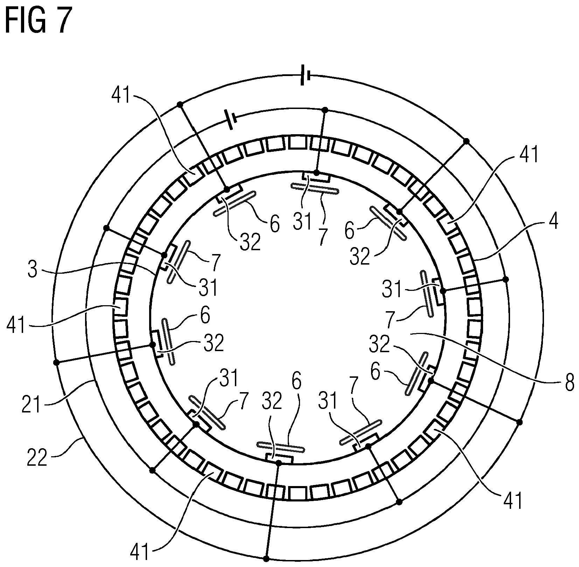

Figur 7 zeigt eine ringförmigen Röntgenquelle 3 und einen Detektorring 4 eines Computertomographie-Systems 1 gemäß einer weiteren Ausführungsform der Erfindung. Diese Ausführungsform kann in wesentlichen Punkten der in Figur 3 gezeigten entsprechen, wird jedoch dadurch weitergebildet, dass die von der zweiten Gruppe von Teilröntgenquellen 32 erzeugte Röntgenstrahlung mit Röntgenfiltern 6 angepasst wird. Dadurch kann die Unterscheidbarkeit der zwei erzeugten Röntgenspektren noch weiter verbessert werden. Optional können auch vor der ersten Gruppe von Teilröntgenquellen 31 jeweils weitere Röntgenfilter 7 vorgesehen sein, welche sich von den Röntgenfiltern 6 der zweiten Gruppe hinsichtlich ihrer Filtereigenschaften unterscheiden. Damit kann eine noch bessere Unterscheidbarkeit der zwei Röntgenspektren erreichbar sein.

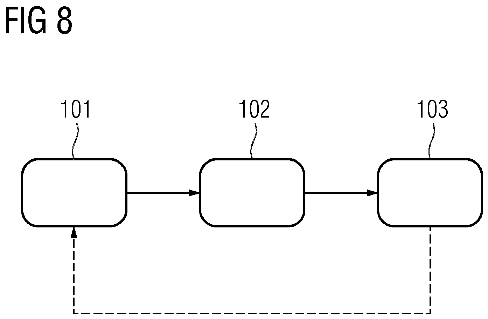

Figur 8 zeigt ein Flussdiagramm eines Verfahrens zum Betreiben eines Computertomographie-Systems 1 gemäß einer Ausführungsform der Erfindung. Dabei werden mehrere Teilröntgenquellen 31, 32 und mehreren Röntgendetektoren 41, die einen Untersuchungsbereich umgeben, örtlich unbeweglich betrieben. In einem ersten Schritt 101 wird eine erste Teilröntgenquelle 31 oder einer ersten Untergruppe von Teilröntgenquellen 31 angesteuert, sodass eine auf den Untersuchungsbereich 8 gerichtete Röntgenstrahlung mit einem ersten Röntgenspektrum erzeugt wird. In einem folgenden Schritt 102 wird eine zweite Teilröntgenquelle 32 oder eine zweite Untergruppe von Teilröntgenquellen 32 angesteuert, sodass eine auf den Untersuchungsbereich 8 gerichtete Röntgenstrahlung mit einem zweiten von dem ersten verschiedenen Röntgenspektrum erzeugt wird. In einem weiteren Schritt 103werden die durch den Untersuchungsbereich 8 gelangten Röntgenstahlen des ersten Röntgenspektrums und des zweiten Röntgenspektrums mit den Röntgendetektoren 41 detektiert. Diese Schritte 101-103 werden vorzugsweise mehrfach für verschiedene Teilröntgenquellen 31, 32 wiederholt, insbesondere bis alle Teilröntgenquellen angesteuert wurden.

## Patentansprüche

1. Computertomographie-System (1) umfassend
eine ringförmige Röntgenquelle (3) mit mehreren kreisförmig oder teilkreisförmig um einen Untersuchungsbereich angeordneten Teilröntgenquellen (31, 32) und einen Detektorring (4) mit mehreren kreisförmig oder teilkreisförmig um den Untersuchungsbereich angeordneten Röntgendetektoren (41),
wobei das Computertomographie-System (1) dazu konfiguriert ist, während eines Computertomographie-Scans bei statischer Röntgenquelle (3) und statischem Detektorring (4) zumindest einige der Teilröntgenquellen (31, 32) einzeln nacheinander anzusteuern und dadurch sowie unter Verwendung der Röntgendetektoren (41) Absorptionsprofile eines Objekts im Untersuchungsbereich aus mehreren Richtungen zu erzeugen,
wobei das Computertomographie-System (1) dazu konfiguriert ist, während des Computertomographie-Scans zumindest ein erstes Röntgenspektrum mit einer ersten Gruppe der Teilröntgenquellen (31) und ein zweites von dem ersten unterscheidbares Röntgenspektrum mit einer zweiten Gruppe der Teilröntgenquellen (32) zu erzeugen und/oder dazu konfiguriert ist, mit den Röntgendetektoren (41) ein Röntgensignal spektral unterscheidbar aufzunehmen.

2. Computertomographie-System (1) gemäß Anspruch 1,
wobei das Computertomographie-System (1) dazu konfiguriert ist, das erste Röntgenspektrum durch Anlegen einer ersten Beschleunigungsspannung bei den Teilröntgenquellen (31) der ersten Gruppe der Teilröntgenquellen (31, 32) und das zweite Röntgenspektrum durch Anlegen einer zweiten Beschleunigungsspannung bei den Teilröntgenquellen (32) der zweiten Gruppe der Teilröntgenquellen (31, 32) zu erzeugen.

3. Computertomographie-System (1) gemäß Anspruch 2,
wobei die ringförmige Röntgenquelle (3) eine erste Spannungsbeschaltung (21) zum Erzeugen einer Beschleunigungsspannung für die ersten Gruppe der Teilröntgenquellen (31, 32) und eine zweite Spannungsbeschaltung (22) zum Erzeugen einer Beschleunigungsspannung für die zweite Gruppe der Teilröntgenquellen (31, 32) umfasst.

4. Computertomographie-System (1) gemäß einem der vorhergehenden Ansprüche,
wobei das Computertomographie-System (1) dazu konfiguriert ist, jeweils abwechselnd zumindest eine der Teilröntgenquellen (31) der ersten Gruppe und zumindest eine der Teilröntgenquellen (32) der zweiten Gruppe anzusteuern.

5. Computertomographie-System (1) gemäß einem der vorhergehenden Ansprüche,
wobei die Teilröntgenquellen (31, 32) auf der ringförmigen Röntgenquelle (3) derart angeordnet sind, dass in Umfangsrichtung jeweils abwechselnd eine Teilröntgenquelle (31) der ersten Gruppe und eine Teilröntgenquelle (32) der zweiten Gruppe angeordnet ist.

6. Computertomographie-System (1) gemäß Anspruch 5,
wobei das Computertomographie-System (1) dazu konfiguriert ist, die Teilröntgenquellen (31, 32) in der Reihenfolge ihrer Anordnung auf dem Kreisumfang anzusteuern.

7. Computertomographie-System (1) gemäß einem der vorhergehenden Ansprüche,
wobei das Computertomographie-System (1) dazu ausgestaltet ist, das zweite Röntgenspektrum zu erzeugen, indem die von der zweiten Gruppe von Teilröntgenquellen (32) erzeugte Röntgenstrahlung mit Röntgenfiltern (6) angepasst wird.

8. Computertomographie-System (1) gemäß Anspruch 7,
wobei das Computertomographie-System (1) dazu ausgestaltet ist, das erste Röntgenspektrum zu erzeugen, indem die von der ersten Gruppe von Teilröntgenquellen (31) erzeugte Röntgenstrahlung ebenfalls mit weiteren Röntgenfiltern (7) angepasst wird,
wobei sich optional die weiteren Röntgenfilter (7) von den Röntgenfiltern (6) der zweiten Gruppe hinsichtlich ihrer Filtereigenschaften unterscheiden.

9. Computertomographie-System (1) gemäß einem der vorhergehenden Ansprüche,
wobei die ringförmige Röntgenquelle (3) zumindest vor der zweiten Gruppe von Teilröntgenquellen (32) Filterslots zum manuellen oder automatischen Einsetzen von Röntgenfiltern (6, 7) umfasst und/oder
wobei die ringförmige Röntgenquelle (3) zumindest vor der zweiten Gruppe von Teilröntgenquellen (32) Röntgenfilter (6, 7) umfasst.

10. Computertomographie-System (1) gemäß einem der vorhergehenden Ansprüche,
wobei das Computertomographie-System (1) dazu konfiguriert ist, während des Computertomographie-Scans eine oder mehrere weitere Röntgenspektren mit einer oder mehreren weiteren Gruppen, darunter zumindest einer dritten Gruppe, der Teilröntgenquellen (31, 32, 33) zu erzeugen.

11. Computertomographie-System (1) gemäß einem der vorhergehenden Ansprüche,
wobei der Detektorring (4) photonenzählende Detektoren umfasst, die dazu konfiguriert sind, zumindest zwei Energieschwellen zu unterscheiden.

12. Computertomographie-System (1) gemäß einem der vorhergehenden Ansprüche,
wobei die Röntgendetektoren (41) als Detektorpaare vorgesehen sind, von denen einer im Wesentlichen nieder-energetische Röntgenstrahlung und der andere im Wesentlichen hochenergetische Röntgenstrahlung absorbiert.

13. Computertomographie-System (1) gemäß einem der Ansprüche 1 bis 12,
wobei die ringförmige Röntgenquelle (3) und der Detektorring (4) jeweils Teilringe sind, die sich gegenüberliegend angeordnet sind, sodass zwischen ihnen der Untersuchungsbereich ist.

14. Computertomographie-System (1) gemäß einem der Ansprüche 1 bis 12,
wobei die ringförmige Röntgenquelle (3) und der Detektorring (4) jeweils über einen vollständigen Ring verteilt sind.

15. Verfahren zum Betreiben eines Computertomographie-Systems (1) mit mehreren Teilröntgenquellen (31, 32) und mehreren Röntgendetektoren (41), die jeweils örtlich unbeweglich betrieben werden und einen Untersuchungsbereich umgeben, umfassend die folgenden Schritte:
(a) Ansteuern einer ersten Teilröntgenquelle (31) oder einer ersten Untergruppe von Teilröntgenquellen (31), sodass eine auf den Untersuchungsbereich gerichtete Röntgenstrahlung mit einem ersten Röntgenspektrum erzeugt wird;
(b) Ansteuern einer zweiten Teilröntgenquelle (32) oder einer zweiten Untergruppe von Teilröntgenquellen (32), sodass eine auf den Untersuchungsbereich gerichtete Röntgenstrahlung mit einem zweiten von dem ersten verschiedenen Röntgenspektrum erzeugt wird;
(c) Detektieren der durch den Untersuchungsbereich gelangten Röntgenstahlen des ersten und des zweiten Röntgenspektrums mit den Röntgendetektoren (41);
wobei vorzugsweise die Schritte a und b zeitversetzt durchgeführt werden.
